# EUROPEAN PATENT SPECIFICATION

(11) **EP 2 689 772 B1**
(45) Date of publication and mention of the grant of the patent: **10.08.2016**
(21) Application number: 12760553.3
(22) Date of filing: 19.03.2012
(51) Int. Cl.: A61K 8/891, A61K 8/34, A61K 8/37, A61K 8/39, A61K 8/73, A61K 8/86, A61K 8/894, A61Q 1/02, A61Q 1/12, A61Q 19/08, A61K 8/02

(54) **SOLID COSMETIC**
KOSMETIKFESTSTOFF
PRODUIT COSMÉTIQUE SOLIDE

(30) Priority: 22.03.2011 JP 2011061991
(43) Date of publication of application: 29.01.2014
(73) Proprietor: Shiseido Company, Ltd., Tokyo 104-0061 (JP)
(72) Inventor: SONOYAMA, Yuji, Yokohama-shi, Kanagawa 224-8558 (JP); CHIHARA, Mariko, Yokohama-shi, Kanagawa 224-8558 (JP)
(74) Representative: Scholz, Volker
(86) International application number: PCT/JP2012/001906
(87) International publication number: WO 2012/127852

(56) References cited:
- EP-A1- 0 374 332
- EP-A2- 1 258 290
- WO-A1-94/27560
- WO-A1-2008/126652
- JP-A- 10 279 424
- JP-A- 57 169 412
- JP-A- 2002 212 033
- JP-A- 2003 063 919
- JP-A- 2007 204 424
- JP-A- 2007 204 424
- JP-A- 2009 137 900
- JP-A- 2009 184 972
- JP-A- 2010 215 614
- JP-A- 2012 001 481
- US-A1- 2003 180 535
- "safe cosmetics, foundations (revised 10/05/2005), Avon Triple Finish Creme-to-Powder Foundation: Color", INTERNET CITATION, 2005, pages 1-6, XP002578971, Retrieved from the Internet: URL:http://www.zerozits.com/safecosmetics/ foundations/htm [retrieved on 2010-01-20]

## Description

### [Technical Field]

The present invention relates to a solid cosmetic material. More particularly, the invention relates to a solid cosmetic material which has a high moisturizing effect, whose caking due to moisture absorption is reduced, and which is superior in stability, usability and sense of use.

### [Background Art]

A conventional solid powder cosmetic material consists of body pigments such as talc, mica, and sericite, color pigments such as iron oxide, as well as oil components, surfactants, fragrances, antioxidants, preservatives and the like, and is marketed as a make-up cosmetic product such as a face powder, foundation, cheek color, and eye shadow, as well as baby powder.

However, such a conventional solid powder cosmetic material has drawbacks such as having no moisturizing property and having a reduced treatment effect on skin. Recently, an increasing number of females suffer from dry skin due to air conditioning and the like. In addition, in view of anti-aging and the like the prevention of skin drying is being focused on, resulting in a demand for the moisturizing property and a moisturizing sense not only in basic cosmetic materials but also in make-up cosmetic materials such as foundation.

Accordingly, attempts were made to add various moisturizing agents to a solid powder cosmetic material such as foundation. However, when an increased amount of a moisturizer component is incorporated to impart a sufficient moisturizing effect to a powder cosmetic material, the resultant product exhibits a problematic deterioration in quality, such as softening of the molded product due to moisture absorption especially when stored for a prolonged period in a humidified environment and reduced strength. This results in an extreme susceptibility to impact due to being dropped and a tendency to be broken easily, as well as dewing occurring on the surface of the molded article. Such problems are attributable to high hygroscopicity possessed naturally by moisturizing agents.

Patent Documents 1 to 3 describe powder cosmetic materials into which moisturizing agents are incorporated. In Patent Document 1, a moisturizing agent is incorporated stably by imparting hydrophobicity to a certain fraction or more of the powder among the total powder content. In Patent Document 2, wetness and freshness are imparted to a skin by incorporating trehalose. In Patent Document 3, a moisturizing effect on skin is achieved by incorporating certain polyethylene glycols, polyoxyethylene methyl glucosides or polyoxypropylene methyl glucosides. Nevertheless, none of such powder cosmetic materials was successful in solving the aforementioned problems experienced after prolonged storage under humidified conditions.

Also in Patent Document 4, it is described that a powder cosmetic material can maintain superior quality even after being stored for a prolonged period in a humidified environment while preserving a moisturizing effect by incorporating certain amounts of baked clay minerals and moisturizing agents therein. Nevertheless, the powder cosmetic material described in Patent Document 4 becomes a powder finish-like product because it contains a large amount of the baked clay minerals, resulting in a problem that it is difficult to realize the moisturizing effect.

While a makeup cosmetic material of a liquid type having a moisturizing property such as a water-in-oil emulsion foundation containing a moisturizing agent has also been developed, a solid makeup cosmetic material which is molded as a solid in an inner tray is in high demand because of the ease of fixing the make up, and it is still highly desired to develop a solid makeup cosmetic material which is superior in moisturizing property, storage stability and actual moisturizing sense.

### [Related Art Documents]

### [Patent Documents]

[Patent Document 1] Japanese Unexamined Patent Publication No. 6(1994)-219925
[Patent Document 2] Japanese Unexamined Patent Publication No. 6(1994)-040845
[Patent Document 3] Japanese Unexamined Patent Publication No. 10(1998)-175823
[Patent Document 4] Japanese Unexamined Patent Publication No. 2008-001686

### [Summary of Invention]

### [Technical Problem]

Under the circumstance mentioned above, an object of the present invention is to provide a solid makeup cosmetic material which has superior stability in a humidified environment while exerting a sufficient moisturizing effect and which allows the moisturizing effect to be actually sensed upon use.

### [Solution to the Problem]

As a result of intensive study to solve the problems described above, we discovered that by allowing the oil component content of a solid cosmetic material to be adjusted to 33% by mass or greater while incorporating 3% by mass or more of a moisturizing agent, it is possible to achieve a sufficient moisturizing effect with reduced moisture absorption in a humidified environment and also to give a feeling of wetness upon use thereby allowing the moisturizing effect to be actually sensed, thus achieving the present invention.

A solid makeup cosmetic material of the present invention comprises 33% by mass or more of an oil component and 3 to 15% by mass of a moisturizing agent with respect to the total amount of the cosmetic material. By incorporating the oil component at a high content, it becomes possible to suppress the moisture absorption even when incorporating 3% by mass or more of the moisturizing agent, and also to give a feeling of wetness upon use thereby allowing the moisturizing effect to be actually sensed.

The moisturizing agent includes but is not limited to glycerin, polyoxyethylene polyoxypropylene dimethyl ether, 1,3-butylene glycol, polyethylene glycol, diglycerin, dipropylene glycol, xylitol, trehalose, erythritol, hyaluronic acid, urea and the like, which may be employed alone or in combination. Especially in view of the moisturizing effect, sense of use and the like, glycerin is preferable as the moisturizing agent.

The solid cosmetic material of the invention preferably contains 10% by mass or more of a silicone-based oil component. By incorporating the silicone-based oil component, the release from the molded article is satisfactory even with the oil component incorporated at a high content, and the usability is also superior with no oiliness or stickiness.

Also the solid cosmetic material of the invention preferably contains 50% by mass or more of a powder. A powder content less than 50% by mass leads to an excessively low hardness which may result in difficulty in molding the material into a solid cosmetic material in an inner tray.

The solid makeup cosmetic material of the invention may be a foundation, for example.

### [Advantageous Effects of Invention]

The solid makeup cosmetic material of the invention has a low hygroscopicity in spite of the moisturizing agent incorporated at an amount of 3% by mass or more, exhibits a high moisturizing effect, undergoes reduced deterioration in quality even in a humidified environment thereby exhibiting superior storage stability, and can exhibit a feeling of wetness upon use which allows the moisturizing effect to be actually sensed.

### [Brief Description of Drawings]

Figure 1 is a graph representing the effect of the oil component content on the hygroscopicity of a solid cosmetic material.
Figure 2 is a graph representing the effect of the moisturizing agent content on the moisturizing effect of a solid cosmetic material.

### [Description of Embodiments]

The solid makeup cosmetic material of the invention contains 33% by mass or more of the oil component with respect to the total amount of the cosmetic material. More preferably, the oil component content is 35% by mass or more, further preferably 37% by mass or more. By incorporating the oil component at 33% by mass or more, moisture absorption can be suppressed even when the moisturizing agent is incorporated at 3% or more, and the moisturizing effect can be actually sensed upon use. While the upper limit of the oil component content is a level capable of imparting a hardness that enables molding into a solid cosmetic material in an inner tray and is not particularly limited. However, it is usually less than 50% by mass. For example, the oil component content, with respect to the total amount of the cosmetic material, is 33% by mass to 47% by mass, more preferably 33% by mass to 45% by mass, and still more preferably 35% by mass to 40% by mass. An oil component incorporated at a level of 50% by mass or more leads to an excessively low hardness which may result in difficulty in molding the material into a solid cosmetic material in an inner tray.

The solid cosmetic material of the invention preferably contains a silicone-based oil component in view of the usability, the sense of use and the like, although no particular limitation is posed. The amount of the silicone-based oil component to be incorporated, with respect to the total amount of the cosmetic material, is preferably 10% by mass or more, more preferably 20% by mass or more, and still more preferably 30% by mass or more. A silicone-based oil content less than 10% by mass tends to result in poor release from the molded article and may exhibit an oily sense of use.

The silicone oil, which may be used in the present invention, is not limited in particular insofar as the effect of the present invention is not undermined. Examples of the silicone oils include linear polysiloxanes such as dimethylpolysiloxane, methylphenylpolysiloxane, diphenylpolysiloxane, and methylhydrogenpolysiloxane; cyclic polysiloxanes such as octamethylcyclotetrasiloxane, decamethylcyclopentasiloxane, dodecamethylcyclohexasiloxane, and tetramethyltetrahydrogencyclotetrasiloxane; various modified polysiloxanes such as amino-modified polysiloxane, polyether-modified polysiloxane, alkyl-modified polysiloxane, and fluorine-modified polysiloxane; silicone resins forming a three-dimensional network structure; silicone rubbers; and the like. In particular, when the linear polysiloxanes or polyether modified polysiloxanes are used, good appliability and good usability are obtained, and further it is more preferable to include linear polysiloxanes having a low viscosity such as a viscosity of 1-50mPa/s.

Other fats and oils, which may be used in the solid cosmetic of the present invention, are not limited in particular insofar as the effect of the present invention is not undermined. Examples of the other fats and oils include liquid fats and oils such as avocado oil, camellia oil, turtle oil, macadamia nut oil, corn oil, a mink oil, olive oil, rapeseed oil, yolk oil, sesame oil, persic oil, wheat germ oil, sasanqua oil, castor bean oil, linseed oil, safflower oil, cottonseed oil, perilla oil, soybean oil, peanut oil, tea seed oil, kaya oil, rice bran oil, Chinese tung oil, Japanese tung oil, jojoba oil, germ oil, triglycerol, glycerol trioctanoate, and glycerol triisopalmitate; solid fats and oils such as cacao butter, coconut oil, horse tallow, hardened coconut oil, palm oil, beef tallow, sheep tallow, hardened beef tallow, palm kernel oil, pig tallow, beef bone tallow, Japanese wax kernel oil, hardened oil, beef leg tallow, Japanese wax, and hardened castor bean oil; waxes such as bees wax, candelilla wax, cotton wax, carnauba wax, bayberry wax, insect wax, whale wax, montan wax, rice bran wax, lanolin, kapok wax, lanolin acetate, liquid lanolin, cane wax, lanolin fatty acid isopropyl ester, hexyl laurate, reduced lanolin, jojoba wax, hard lanolin, shellac wax, POE lanolin alcohol ether, POE lanolin alcohol acetate, POE cholesterol ether, lanolin fatty acid polyethylene glycol, and POE hydrogenated lanolin alcohol ether; hydrocarbon oils such as liquid paraffin, ozokerite, squalane, pristane, paraffin, ceresin, squalene, vaseline, and micro-crystalline wax; and synthetic ester oils such as isopropyl myristate, cetyl octanoate, octyldodecyl myristate, isopropyl palmitate, butyl stearate, hexyl laurate, myristyl myristate, decyl oleate, hexyldecyl dimethyl octanoate, cetyl lactate, myristyl lactate, lanolin acetate, isocetyl stearate, isocetyl isostearate, cholesteryl 12-hydroxy stearate, ethylene glycol di-2-ethyl hexanoate, a dipentaerythritol fatty acid ester, N-alkyl glycol mono-isostearate, neopentyl glycol dicaprate, diisostearyl malate, glycerol di-2-heptyl undecanoate, trimethylolpropane tri-2--ethyl hexanoate, trimethylolpropane triisostearate, pentaerythritol tetra-2-ethyl hexanoate, glycerol tri-2-ethyl hexanoate, trimethylolpropane triisostearate, cetyl-2-ethyl hexanoate, 2-ethyl hexyl palmitate, glycerol trimyristate, tri-2-heptylundecanoic acid glyceride, a castor oil fatty acid methyl ester, oleyl oleate, cetostearyl alcohol, acetoglyceride, 2-heptyl undecyl palmitate, diisobutyl adipate, an N-lauroyl-L-glutamic acid-2-octyl dodecyl ester, di-2-heptyl undecyl adipate, ethyl laurate, di-2-ethyl hexyl sebacate, 2-hexyl decyl myristate, 2-hexyl decyl palmitate, 2-hexyl decyl adipate, diisopropyl sebacate, 2-ethyl hexyl succinate, ethyl acetate, butyl acetate, amyl acetate, and triethyl citrate; and the like.

The solid cosmetic material of the invention contains 3 to 15% by mass of a moisturizing agent with respect to the total amount of the cosmetic material. More preferably, the amount of the moisturizing agent to be incorporated is 3 to 10% by mass, and still more preferably 5 to 10% by mass. An amount of the incorporated moisturizing agent less than 3% by mass leads to difficulty in obtaining a sufficient moisturizing effect, while an amount exceeding 15% by mass leads to high hygroscopicity, resulting in a tendency for poor storage stability and poor release from the molded article upon abrasion.

In the invention, a moisturizing agent refers to one exhibiting a % mass change of 0.2% to 30% when 5 g of the moisturizing agent component is weighed accurately into a 20 mL screw tube which is then allowed to stand night and day at 37°C in a relative humidity of 98%. By using such a moisturizing agent component, a solid makeup cosmetic material which has a high moisturizing effect and which is superior in usability and sense of use without exhibiting dustiness or stickiness can be prepared.

The moisturizing agent employed preferably in the invention which satisfies the aforementioned conditions includes, but is not limited to, glycerin, polyoxyethylene polyoxypropylene dimethyl ether, 1,3-butylene glycol, polyethylene glycol, diglycerin, dipropylene glycol, xylitol, trehalose, erythritol, hyaluronic acid, urea and the like, one of which or a combination of two or more selected from which may be employed. Especially in view of a high moisturizing effect, sense of use and the like, glycerin is preferable as the moisturizing agent.

Table 1 shown below indicates the hygroscopicity values of exemplary moisturizing agent components which are preferably employed in the present invention and were measured by the aforementioned method. Each material was measured twice by the aforementioned method and the mass (g) increased by moisture absorption, the % increase in mass (%) and the mean value (%) of the % increase in mass are indicated.

**[Table 1]**

| Ingredients | Increase in Mass (g) | % Increase in Mass (%) | Mean Value (%) |
|---|---|---|---|
| Glycerin | 0.40 | 7.8 | |
| | 0.40 | 7.8 | 7.8 |
| Polyoxyethylene (9) polyoxypropylene (2) dimethyl ether | 0.29 | 5.8 | |
| | 0.29 | 5.7 | 5.7 |
| Polyoxyethylene (14) polyoxypropylene (7) dimethyl ether | 0.24 | 4.7 | |
| | 0.24 | 4.6 | 4.6 |
| Polyoxyethylene (36) polyoxypropylene (41) dimethyl ether | 0.17 | 3.3 | |
| | 0.18 | 3.5 | 3.4 |
| Polyoxyethylene (55) polyoxypropylene (28) dimethyl ether | 0.21 | 4.2 | |
| | 0.23 | 4.5 | 4.3 |
| 1,3-Butylene glycol | 0.50 | 10.2 | |
| | 0.50 | 9.8 | 10.0 |
| Polyethylene glycol 400 | 0.40 | 8.0 | |
| | 0.36 | 7.1 | 7.6 |
| Diglycerin | 0.58 | 11.2 | |
| | 0.61 | 11.9 | 11.5 |
| Dipropylene glycol | 1.03 | 18.4 | |
| | 1.00 | 19.1 | 18.8 |
| Xylitol | 0.52 | 10.3 | |
| | 0.65 | 13.0 | 11.7 |
| Trehalose | 0.04 | 0.9 | |
| | 0.06 | 1.1 | 1.0 |
| Erythritol | 0.10 | 2.0 | |
| | 0.20 | 4.1 | 3.0 |
| Hyaluronic acid Na | 0.78 | 16.3 | |
| | 0.81 | 17.2 | 16.7 |
| Urea | 0.29 | 5.9 | |
| | 0.51 | 10.1 | 8.0 |

The solid cosmetic material of the invention contains a powder in addition to the aforementioned oil component and moisturizing agent. While the amount of the powder to be incorporated is not limited as long as the effect of the invention can be achieved, it is usually 50% by mass or more with respect to the total amount of the cosmetic material, and more preferably 55% by mass or more. An amount of the incorporated powder less than 50% by mass leads to an excessively low hardness which may result in a difficulty in molding the material into a solid cosmetic material in an inner tray, and makes it difficult to take the molded material up using a brush, sponge and the like. The upper limit of the amount of the powder to be incorporated is the remainder after subtracting the amount of the aforementioned oil component and moisturizing agent to be incorporated.

The powder components, which may be used in the present invention, are not limited in particular insofar as the effect of the present invention is not undermined. Examples of the powder include extender pigments such as talc, sericite, mica, kaolin, magnesium carbonate, calcium carbonate, aluminum silicate, barium silicate, calcium silicate, magnesium silicate, strontium silicate, a tungstic acid metal salt, magnesium, silica, zeolite, barium sulfate, calcined calcium sulfate, calcium phosphate, fluorine-apatite, hydroxyapatite, metallic soaps, boron nitride nylon powder, polyethylene powder, cellulose powder, silicone powder, and zinc oxide; colorants such as carbon black, titanium oxide, zinc oxide, ultramarine blue, iron blue, chromium oxide, an organic tar-based pigment and lake; complex pigments such as titanated mica, and iron oxide coated titanated mica; and these powder components which are hydrophobically treated with a silicone compound, a fluorine-modified silicone compound, a fluorine compound, a higher fatty acid, a higher alcohol, a fatty acid ester, a metal soap, an amino acid, a quaternary ammonium salt, an alkylphosphate, or the like.

As the method of hydrophobic treatment of powder, any method, which can hydrophobize a powder, may be used. The method is not particularly limited, and ordinary surface treatment methods such as the vapor phase method, liquid phase method, autoclave method, mechanochemical method, etc., may be used, for example.

For example, when the treatment is carried out by adding a hydrophobizing agent to a raw material powder, the hydrophobizing agent may be diluted in a suitable solvent (e.g., dichloromethane, chloroform, hexane, ethanol, xylene, volatile silicone, etc.) and then added, or may be directly added. To agitate and mix the powder and the hydrophobizing agent, a ball mill, faujasite ball mill, vibration ball mill, attritor, pot mill, rod mill, pan mill, homo-mixer, homodisperser, Henschel mixer, Nauta mixer, etc., may be used. In addition, the method for polymerizing a cyclic organosiloxane on the powder surface at a low temperature of 100°C. or less by a vapor phase reaction utilizing the activity of the powder surface (see Japanese Examined Patent Publication (Kokoku) No. 1(1989)-054380), the method of adding pendant groups such as glyceryl-α-monoallylether to the Si-H portions of the silicone polymer of the surface after the above method (see Japanese Examined Patent Publication (Kokoku) No. 1 (1989)-054381), etc., may be used.

Examples of the hydrophobically treated powders include a fatty acid dextrin treated powder; trimethylsiloxysilicate treated powder; fluorine-modified trimethylsiloxysilicate treated powder; methylphenylsiloxysilicate treated powder; fluorine-modified methylphenylsiloxysilicate treated powder; powders treated with a low viscosity to high viscosity oily polysiloxane such as dimethyl polysiloxane, diphenyl polysiloxane, or methylphenyl polysiloxane; gum-like polysiloxane treated powder; methylhydrogen polysiloxane treated powder; fluorine-modified methylhydrogen polysiloxane-treated powder; powders treated with an organic silyl compound such as methyl trichlorosilane, methyl trialkoxysilane, hexamethyl disilane, dimethyl dichlorosilane, dimethyl dialkoxysilane, trimethyl chlorosilane, trimethyl alkoxysilane, or fluorine-substituent thereof; powders treated with an organic modified silane such as ethyl trichlorosilane, ethyl trialkoxysilane, propyl trichlorosilane, propyl trialkoxysilane, hexyl trichlorosilane, hexyl trialkoxysilane, long-chain alkyl trichlorosilane, and long-chain alkyl triethoxysilane, or fluorine substituent thereof; amino-modified polysiloxane treated powder; fluorine-modified polysiloxane treated powder; fluoroalkyl phosphoric acid treated powder; fluoroalkyl phosphate ester treated powder; and the like.

While the solid powder material of the invention can be produced by a "dry molding method" in which the powder component and the oily component are mixed and molded without using a solvent, it is produced preferably by a "wet molding method" detailed below. The molded material can be taken up by abrasion using a sponge and the like in an extremely satisfactory manner when produced by the wet molding method compared with a conventional dry molding method.

For example, the powder component is mixed first using a Henschel mixer or the like, and then the oily component containing the moisturizing agent is admixed uniformly to prepare a cosmetic material base. Then this cosmetic material base is mixed with a solvent to form a slurry. Then this slurry is filled in a container. If the slurry is poorly spread in the container upon filling, it can be filled uniformly by imparting gentle vibrations to a degree that the filled material is not spilled. After the container is filled, the solvent is removed for solidification. The removal of the solvent can be accomplished by a standard method, such as allowing to stand for drying, drying with warming, drying with a warm air flow, and vacuum drying. The aforementioned production method is referred to as a wet molding method, the details of which are described in Examined Japanese Patent Publications Nos. 57(1982)-060004, 61(1986)-054766 and the like.

While the amount of the solvent may vary depending on the composition of the powder component and the amount of the oily component to be incorporated, it is preferable to make the viscosity that at which the deaeration of the slurry and the filling into the container and the like can readily be accomplished, and an amount 0.5 to 1.5 times (by weight) that of the cosmetic material base is generally preferred. An excessively large amount of the solvent leads to a prolonged amount of time required for drying and results in cracks or reduced content after drying, as well as reduced impact resistance.

The solvent employed in the invention may for example be water or an alcohol such as methanol, ethanol, and isopropyl alcohol, benzene, toluene, THF, paraffin, and silicone, and one or a mixture of two or more of the above may be employed depending on the characteristics of the powder component and the oily component employed. Among these, ethanol is particularly preferred.

Since the oily component and the like may be removed together with the solvent when the production employs a wet molding method, increased amounts relative to the intended contents in the final product should be incorporated during the production. Since the level to which the amount should be increased may vary depending on the type and the amount of the components to be incorporated and the solvent, production conditions, etc. it may be appropriately determined, and it is preferable in most of the cases to increase the amount to 120 to 150% by mass, and in case of a high compatibility with the solvent it may be required to increase the amount up to about 200% by mass.

If necessary, the cosmetic in accordance with the present invention may also contain other arbitrary ingredients which are ordinarily used in cosmetics, such as surfactants, thickeners, agents such as vitamins, perfumes, anti-oxidants, and antiseptics and fungicides, within a range such that the effects of the present invention may not be affected adversely in addition to the essential ingredients described above.

Examples of lipophilic nonionic surfactants include, but are not particularly restricted to, sorbitan fatty acid esters such as sorbitan monoisostearate, sorbitan monolaurate, sorbitan monopalmitate, sorbitan monostearate, sorbitan sesquioleate, sorbitan trioleate, diglycerol sorbitan penta-2-ethylhexanoate, and diglycerol sorbitan tetra-2-ethylhexanoate; (poly)glycerol esters of fatty acids such as a glycerin cotton seed oil fatty acid ester, glyceryl monoerucate, glyceryl sesquioleate, glyceryl monostearate, glyceryl α, α'-oleate pyroglutamate, and glyceryl monostearate malate; propylene glycol fatty acid esters such as propylene glycol monostearate; hardened castor oil derivatives; glycerin alkyl ethers; and the like.

Examples of hydrophilic nonionic surfactants include, but are not particularly restricted to, POE sorbitan fatty acid esters such as POE-sorbitan monooleate, POE-sorbitan monostearate, POE-sorbitan monooleate, and POE-sorbitan tetraoleate; POEsorbitol fatty acid esters such as POE-sorbitol monolaurate, POE-sorbitol monooleate, POE-sorbitol pentaoleate, and POE-sorbitol monostearate; POE glycerol fatty acid esters such as POE-glyceryl monostearate, POE-glyceryl monoisostearate, and POE-glyceryl triisostearate; POE fatty acid esters such as POE monooleate, POE distearate, POE monodioleate, and ethylene glycol distearate; POE alkyl ethers such as POE lauryl ether, POE oleyl ether, POE stearyl ether, POE behenyl ether, POE2-octyldodecyl ether, and POE cholestanol ether; POE alkylphenyl ethers such as POE octylphenyl ether, POE nonylphenyl ether, and POE dinonylphenyl ether; pluaronic types such as Pluronic; POE-POP alkyl ethers such as POE-POP cetyl ether, POE-POP 2-decyl tetradecyl ether, POE-POP monobutyl ether, POE-POP hydrogenated lanolin, and POE-POP glycerol ether; tetra POE-tetra POP ethylenediamine condensates such as Tetronic; POE castor oil or hydrogenated castor oil derivatives such as POE castor oil, POE hydrogenated castor oil, POE hydrogenated castor oil monoisostearate, POE hydrogenated castor oil triisostearate, POE hydrogenated castor oil monopyroglutamate monoisostearic acid diester, and POE hydrogenated castor oil maleate; POE beeswax-lanolin derivatives such as POE sorbitol beeswax; alkanolamides such as coconut oil fatty acid diethanolamide, lauric acid monoethanolamide, and fatty acid isopropanolamide; POE propylene glycol fatty acid esters; POE alkylamines; POE fatty acid amides; sucrose fatty acid esters; POE nonylphenyl formaldehyde condensates; alkyl ethoxydimethylamine oxides; trioleyl phosphate; and the like.

Examples of the vitamins include vitamin A and derivatives thereof such as vitamin A palmitate, vitamin B and derivative thereof such as pyridoxine hydrochloride, vitamin C and derivative thereof such as L-ascorbic acid, L-ascorbyl sulfate, and ascorbyl phosphate, vitamin D and derivatives thereof such as cholecalciferol and ergocalciferol, vitamin E and derivatives thereof such as δ-tocopherol and vitamin E acetate, etc.

Examples of the ultraviolet absorber include benzoic acid-based ultraviolet absorbers such as para-aminobenzoic acid (hereinafter, abbreviated to PABA), PABA monoglycerin ester, N,N-dipropoxy PABA ethyl ester, N,N-diethoxy PABA ethyl ester, N,N-dimethyl PABA ethyl ester, N,N-dimethyl PABA butyl ester, and N,N-dimethyl PABA methyl ester; anthranilic acid-based ultraviolet absorbers such as homomenthyl-N-acetyl anthranilate; salicylic acid-based ultraviolet absorbers such as amyl salicylate, menthyl salicylate, homomenthyl salicylate, octyl salicylate, phenyl salicylate, benzyl salicylate, and p-isopropanolphenyl salicylate; cinnamic acid-based ultraviolet absorbers such as octyl methoxycinnamate, ethyl-4-isopropyl cinnamate, methyl-2,5-diisopropyl cinnamate, ethyl-2,4-diisopropyl cinnamate, methyl-2,4-diisopropyl cinnamate, propyl-p-methoxycinnamate, isopropyl-p-methoxycinnamate, isoamyl-p-methoxycinnamate, octyl-p-methoxycinnamate (2-ethylhexyl-p-methoxycinnamate), 2-etnoxyethyl-p-methoxycinnamate, cyclohexyl-p-methoxycinnamate, ethyl-α-cyano-β-phenyl cinnamate, 2-ethylhexyl-α-cyano-β-phenyl cinnamate, and glyceryl mono-2-ethylhexanoyl di-para-methoxycinnamate; 3-(4'-methylbenzylidene)-d,1-camphor; 3-benzylidene-d,1-camphor; urocanic acid; urocanic acid ethyl ester; 2-phenyl-5-methylbenzoxazole; 2,2'-hydroxy-5-methylphenylbenzotriazole; 2-(2'-hydroxy-5'-t-octylphenyl)benzotriazole; 2-(2'-hydroxy-5'-methylphenylbenzotriazole; dibenzalazine; dianisoylmethane; 4-methoxy-4'-t-butyldibenzoylmethane; 5-(3,3-dimethyl-2-norbornylidene)-3-pentan-2-one; and the like.

Examples of the antiseptic include methyparaben, ethylparaben, propylparaben, butylparaben, phenoxyethanol, N-coconut oil fatty acid acyl L-arginine ethyl DL-pyrrolidonecarbaxylate, sodium dehydroacetate, ethylhexylglycerin, (silver/zinc/ammonium) zeolite, etc.

The solid makeup cosmetic material of the present invention is a soft type solid powder cosmetic material, and preferably is a non-aqueous powder cosmetic material containing substantially no water. It can be applied to various forms of the cosmetic materials in which large amounts of powders are incorporated, for example, a powdery foundation, pressed powder, eye shadow, loose powder, and cheek color.

### [Examples]

The present invention will be described in detail with reference to the Examples shown below. However, the present invention is not limited to these Examples. While the following Examples employed glycerin as a moisturizing agent, similar effects can be achieved with other moisturizing agents. Unless otherwise specified, the amount of an incorporated component is represented as a % by mass with respect to the system to which it was incorporated.

### Investigation of amount of oil component to be incorporated Preparation of solid cosmetic material

The solid cosmetic materials having different amounts of the incorporated oil components indicated in Table 2 shown below were prepared by a wet molding method according to the method described above. First, the powder components are mixed using a Henschel mixer or the like, and thereafter the oily components including the moisturizing agent were added and mixed uniformly to prepare a cosmetic material base. Then, this cosmetic material base was mixed with ethanol as a solvent to form a slurry. This slurry was filled in a container and the solvent was removed to form a solid. The removal of the solvent was conducted using a 55°C dryer.

### Evaluation of stability under humidified condition

After allowing to stand for 2 weeks in an environment at 37°C and 98% relative humidity, the hygroscopicity and the appearance stability were evaluated. The hygroscopicity was calculated as a % increase in mass (%) based on the initial value (mass before starting the test) by measuring the mass of a sample before and 1 week and 2 weeks after starting the test.

The appearance stability was evaluated by visual inspection of the appearance after 2 weeks according to the following evaluation criteria:
○: No problem
x: Problems such as "dewing", "swelling", and "cracking" occurred.

### Evaluation of actual moisturizing sense

To evaluate the actual moisturizing sense, 10 specialized panelists actually used each sample and the actual moisturizing sense was judged based on the number of panelists who sensed the moisturizing effect according to the following criteria.
⊚: 6 panelists or more
⊙: 4 to 5 panelists
Δ: 2 to 3 panelists
X: 0 to 1 panelists

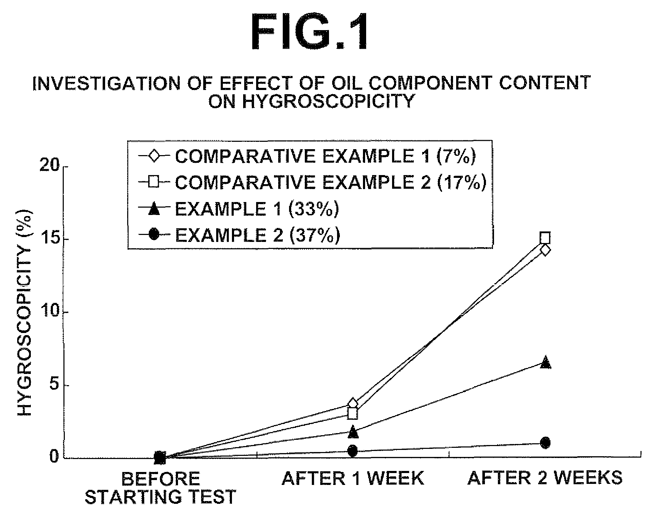

The results are indicated in Table 2 shown below and Figure 1.

**[Table 2]**

| | Component | Cemparative 1 | Comparative 2 | Example 1 | Example 2 |
|---|---|---|---|---|---|
| Oil component | Dimethyl polysiloxane (6mPa.s) | 5 | 15 | 31 | 35 |
| | Polyoxyethylene/methyl polysiloxane copolymer¹ | 1 | 1 | 1 | 1 |
| | Ethylhexyl methoxycinnamiate | 1 | 1 | 1 | 1 |
| Moisturizing agent | Glycerin | 5 | 5 | 5 | 5 |
| Powder | Hydrophobically modified mica | 25 | 25 | 25 | 25 |
| | Pigment-grade titanium oxide | 8 | 8 | 8 | 8 |
| | Finely divided titanium oxide | 3 | 3 | 3 | 3 |
| | Zinc oxide | 2 | 2 | 2 | 2 |
| | Hydrophobically modified iron oxide (red) | 0.31 | 0.31 | 0.31 | 0.31 |
| | Hydrophobically modified iron oxide (yellow) | 0.84 | 0.84 | 0.84 | 0.84 |
| | Hydrophobically modified iron oxide (black) | 0.08 | 0.08 | 0.08 | 0.08 |
| | (Vinyl dimethicone/methicone silsesquioxane) crosspolymer | 4 | 4 | 4 | 4 |
| | Chlorphenesin | 0.2 | 0.2 | 0.2 | 0.2 |
| | Hydrophobically modified talc | Remainder | Remainder | Remainder | Remainder |
| | Total | 100 | 100 | 100 | 100 |
| | Total of oil components | 7 | | 33 | 37 |
| | Hygroscopicity (%) after 1 week | 4.1 | 3.5 | 2.2 | 0.9 |
| | Hygroscopicity (%) after 2 weeks | 14.6 | 15.4 | 7.0 | 1.4 |
| | Appearance stability | x | x | O | O |
| | Actual moisturizing sense | x | Δ | ○ | ⊚ |

| | | | | | |
|---|---|---|---|---|---|
| *1: Silicone KF6017 (manufactured by Shin-Etsu Chemical Co., Ltd.) | | | | | |

Each of Examples 1 and 2 whose oil component contents are 33% by mass and 37% by mass respectively exhibited low hygroscopicity even when containing 5% by mass of glycerin, and did not exhibit a problem such as dewing or caking even after a prolonged storage under a humidified condition, and had superior appearance stability. IN contrast, each of Comparative Examples 1 and 2 whose oil component contents are 7% by mass and 17% by mass respectively exhibited high hygroscopicity, and exhibited dewing or caking after storage for 2 weeks under humidified conditions.

Also, the amount of the incorporated moisturizing agent was equal at 5% by mass in each sample. Each of Examples 1 and 2 having a high oil component content allowed the moisturizing effect to be actually sensed upon use, while each of Comparative Examples 1 and 2 having a low oil component content could not allow the moisturizing effect to be actually sensed at a satisfactory level. Investigation of amount of moisturizin agent to be incorporated

The solid cosmetic materials having different amounts of the incorporated moisturizing agent indicated in Table 3 shown below were prepared by a wet molding method similar to that described above.

**[Table 3]**

| | Component | Comparative 3 | Comparative 4 | Example 3 | Example 4 | Example 5 |
|---|---|---|---|---|---|---|
| Oil component | Diisostearyl malate | 4 | 4 | 4 | 4 | 4 |
| | Dimethyl polysiloxane (6mPa·s) | 32 | 31 | 29 | 27 | 27 |
| | Polyoxyethylene/methyl polysiloxane copolymer*1 | 1 | 1 | 1 | 1 | 1 |
| | Ethylhexyl methoxycinnamiate | 1 | 1 | 1 | 1 | 1 |
| Moisturizing agent | Glycerin | - | 1 | 3 | 5 | 10 |
| Powder | Hydrophobically modified mica | 25 | 25 | 25 | 25 | 25 |
| | Pigment-grade titanium oxide | 8 | 8 | 8 | 8 | 8 |
| | Finely divided titanium oxide | 3 | 3 | 3 | 3 | 3 |
| | Zinc oxide | 2 | 2 | 2 | 2 | 2 |
| | Hydrophobically modified iron oxide (red) | 0.31 | 0.31 | 0.31 | 0.31 | 0.31 |
| | Hydrophobically modified iron oxide (yellow) | 0.84 | 0,84 | 0.84 | 0.84 | 0.84 |
| | Hydrophobically modified iron oxide (black) | 0.08 | 0.08 | 0.08 | 0.08 | 0.08 |
| | (Vinyl dimethicone/methicone silsesquioxane) crosspolymer | 4 | 4 | 4 | 4 | 4 |
| | Chlorphenesin | 0.2 | 0.2 | 0.2 | 0.2 | 0.2 |
| | Hydrophobically modified talc | Remainder | Remainder | Remainder | Remainder | Remainder |
| | Total | 100 | 100 | 100 | 100 | 100 |
| | Moisturizing effect | × | × | ○ | ○ | ⊚ |
| | Appearance stability | ○ | ○ | ○ | ○ | ○ |
| | Actual moisturizing sense | × | × | ○ | ○ | ⊚ |

| | | | | | | |
|---|---|---|---|---|---|---|
| *1: Silicone KF6017 (manufactured by Shin-Etsu Chemical Co., Ltd.) | | | | | | |

### Evaluation of moisturizing effect

The water content of the corneal layer (electrical capacitance (a.u.)) was measured using a skin water level measurement machine (Corneometer CM825) [Courage + Khazaka Electronic, Co.].

The arms of 10 volunteers were washed with solid soaps, allowed to stand for 15 minutes in a thermostat controlled highly humid room and subjected to the measurement of the water content of the corneal layers of the inner sides of the arms (measurement 1). Then, a lotion having no moisturizing effect was applied to the inner sides of the arms, and thereafter each sample was applied to a discrete position and allowed to stand for 4 hours. The sample was wiped off 8 times using tissue papers, and after allowing to stand for 15 minutes the position to which each sample was applied was subjected to the measurement of the water content of the corneal layer (measurement 2). As a control, similar measurements were conducted without applying the samples. The values obtained by subtracting measurement 1 from measurement 2 were the calculated amounts of the increase in water content of the corneal layer (a.u.). The significant difference test was conducted by the T test, and a significant difference was judged to be present at p<0.05.

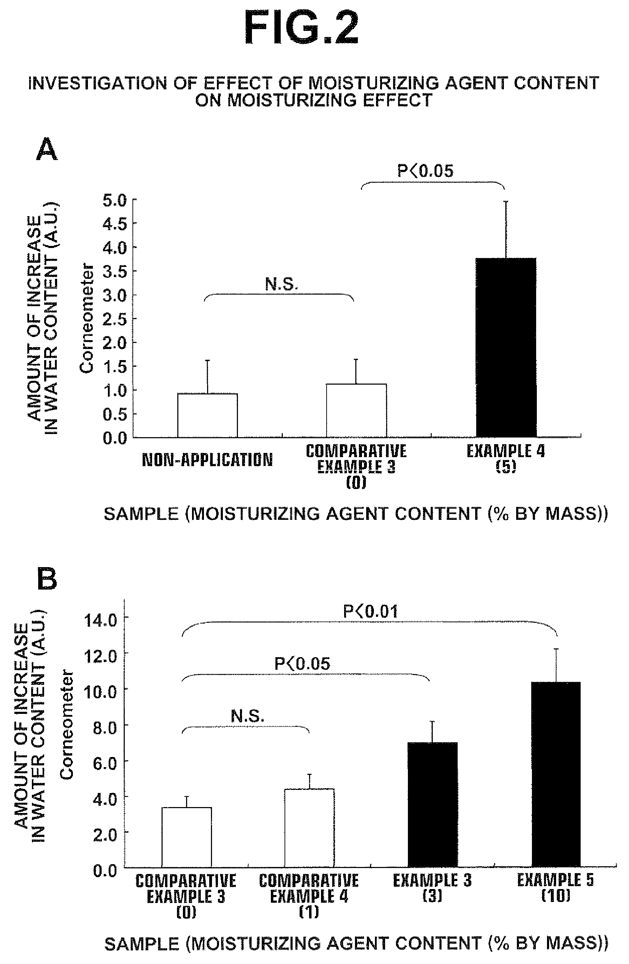

The results are shown in Figure 2.

A of Figure 2 shows the increase in water content of the corneal layer (a.u.) when the control group (non-application), Comparative Example 3 and Example 4 were tested simultaneously on the same volunteers (n=10). Comparative Example 3 containing no moisturizing agent exhibited a degree similar to that in the control group, and had no significant difference (N.S.). Example 4 containing the moisturizing agent at 5% by mass increased the water content of the corneal layer significantly when compared with Comparative Example 3 containing no moisturizing agent (p<0.05).

B of Figure 2 shows the increase in water content of the corneal layer (a.u.) when Comparative Examples 3 and 4 and Examples 3 and 5 having the moisturizing agent contents of 0, 1, 3 and 10% by mass, respectively were tested simultaneously on the same volunteers (n=10). Even in Example 3 containing 3% by mass of the moisturizing agent, a significant increase in water content of the corneal layer was achieved when compared with Comparative Example 3 containing no moisturizing agent (p<0.05).

Based on the significant difference in the increase in water content of the corneal layer described above when compared with Comparative Example 3 (containing no moisturizing agent), the moisturizing effect was judged according to the following criteria and the results are shown in Table 3.
⊚: Significantly different at p<0.01
○: Significantly different at p<0.05
x: Not significantly different at p>0.05

Also, the appearance stability under the humidified condition and the actual sense of use were evaluated in the same manner as that described above, and the results are shown in Table 3.

As evident from Table 3, any of the solid cosmetic materials of Examples 3 to 5 containing 33% by mass or more of the oil components and 3% by mass or more of the moisturizing agents exhibited superior moisturizing effect, storage stability and actual moisturizing sense.

The formulation examples of the solid makeup cosmetic materials of the invention are shown below as Examples. The amounts incorporated are represented as % by mass with respect to the total amount of the cosmetic material, and the preparations were prepared by the wet molding method as described above.

### Example 6: Powdery foundation

| Component | Incorporated amount (%by mass) |
|---|---|
| Diisostearyl malate | 4 |
| Dimethyl polysiloxane (6mPa·s) | 29 |
| Polyoxyethylene/methylpolysiloxane copolymer *1 | 1 |
| Methoxycinnamic acid | 1 |
| Polyoxyethylene (9) polyoxypropylene (2) dimethyl ether | 5 |
| Hydrophobically modified mica | 25 |
| Pigment grade titanium oxide | 8 |
| Finely divided titanium oxide | 3 |
| Zinc oxide | 2 |
| Hydrophobically modified iron oxide (red) | 0.31 |
| Hydrophobically modified iron oxide (yellow) | 0.84 |
| Hydrophobically modified iron oxide (black) | 0.08 |
| (Vinyl dimethicone/methicone silsesquioxane) crosspolymer | |
| | 4 |
| Chlorphenesin | 0.2 |
| Hydrophobically modified talc | Remainder |
| Total | 100 |

### Example 7: Powdery foundation

| Component | Incorporated amount (%by mass) |
|---|---|
| Diisostearyl malate | 4 |
| Dimethyl polysiloxane (6mPa·s) | 29 |
| Polyoxyethylene/methylpolysiloxane copolymer *1 | 1 |
| Ethylhexyl methoxycinnamiate | 1 |
| 1,3-Butylene glycol | 7 |
| Hydrophobically modified mica | 25 |
| Pigment grade titanium oxide | 8 |
| Finely divided titanium oxide | 3 |
| Zinc oxide | 2 |
| Hydrophobically modified iron oxide (red) | 0.31 |
| Hydrophobically modified iron oxide (yellow) | 0.84 |
| Hydrophobically modified iron oxide (black) | 0.08 |
| (Vinyl dimethicone/methicone silsesquioxane) crosspolymer | |
| | 4 |
| Chlorphenesin | 0.2 |
| Hydrophobically modified talc | Remainder |
| Total | 100 |

### Example 8: Powdery foundation

| Component | Incorporated amount (% by mass) |
|---|---|
| Diisostearyl malate | 4 |
| Dimethyl polysiloxane (6mPa·s) | 29 |
| Polyoxyethylene/methylpolysiloxane copolymer *1 | 1 |
| Ethylhexyl methoxycinnamiate | 1 |
| Sodium hyaluronate | 10 |
| Hydrophobically modified mica | 25 |
| Pigment grade titanium oxide | 8 |
| Finely divided titanium oxide | 3 |
| Zinc oxide | 2 |
| Hydrophobically modified iron oxide (red) | 0.31 |
| Hydrophobically modified iron oxide (yellow) | 0.84 |
| Hydrophobically modified iron oxide (black) | 0.08 |
| (Vinyl dimethicone/methicone silsesquioxane) crosspolymer | 4 |
| Chlorphenesin | 0.2 |
| Hydrophobically modified talc | Remainder |
| Total | 100 |

The solid makeup cosmetic materials of the Examples described above exhibited superior moisturizing effect, storage stability and actual moisturizing sense.

## Claims

1. A solid makeup cosmetic material comprising, based on the total amount of the cosmetic material, 33 to 45% by mass of an oil component, 3 to 15% by mass of a moisturizing agent and 50% by mass or more of a powder, wherein the solid makeup cosmetic material is a non-aqueous powder cosmetic material.

2. The solid makeup cosmetic material according to Claim 1 wherein the moisturizing agent is one or more selected from glycerin, polyoxyethylene polyoxypropylene dimethyl ether, 1,3-butylene glycol, polyethylene glycol, diglycerin, dipropylene glycol, xylitol, trehalose, erythritol, hyaluronic acid, and urea.

3. The solid makeup cosmetic material according to Claim 1 or 2 wherein the moisturizing agent comprises glycerin.

4. The solid makeup cosmetic material according to any one of Claims 1 to 3 wherein 10% by mass or more of a silicone-based oil component is contained based on the total amount of the cosmetic material.

5. The solid makeup cosmetic material according to any one of Claims 1 to 4 which is a foundation.

## Patentansprüche

1. Festes Make-up-Kosmetikmaterial umfassend, basierend auf der Gesamtmenge des Kosmetikmaterials, 33 bis 45 Massenprozent einer Ölkomponente, 3 bis 15 Massenprozent eines Feuchtigkeitsspenders und 50 Massenprozent oder mehr eines Pulvers, wobei das feste Make-up-Kosmetikmaterial ein nicht-wässriges Pulverkosmetikmaterial ist.

2. Festes Make-up-Kosmetikmaterial nach Anspruch 1, wobei der Feuchtigkeitsspender eines oder mehrere ist, ausgewählt aus Glyzerin, Polyoxyethylen Polyoxypropylendimethylether, 1,3-Butylenglykol, Polyethylenglykol, Diglyzerin, Dipropylenglykol, Xylitol, Trehalose, Erythrithol, Hyaluronsäure und Harnstoff.

3. Festes Make-up-Kosmetikmaterial nach Anspruch 1 oder 2, wobei der Feuchtigkeitsspender Glyzerin umfasst.

4. Festes Make-up-Kosmetikmaterial nach einem der Ansprüche 1 bis 3, wobei 10 Massenprozent oder mehr einer Ölkomponente auf Silikonbasis enthalten sind, basierend auf der gesamten Menge des Kosmetikmaterials.

5. Festes Make-up-Kosmetikmaterial nach einem der Ansprüche 1 bis 4, welches eine Basis ist.

## Revendications

1. Produit cosmétique solide comprenant, par rapport à la quantité totale du produit cosmétique, 33 à 45 % en masse d'un composant huileux, 3 à 15 % en masse d'un agent hydratant et 50 % en masse ou plus d'une poudre, le produit cosmétique solide de maquillage étant un produit cosmétique en poudre non aqueuse.

2. Produit cosmétique solide de maquillage selon la Revendication 1 dans lequel l'agent hydratant est constitué d'un ou de plusieurs composés sélectionné(s) parmi la glycérine, l'éther de diméthyle polyoxyéthyléné polyoxypropyléné, le 1-3-butylène glycol, le polyéthylène glycol, la diglycérine, le dipropylène glycol, le xylitol, le tréhalose, l'érythritol, l'acide hyaluronique et l'urée.

3. Produit cosmétique solide de maquillage selon la Revendication 1 ou la Revendication 2 dans lequel l'agent hydratant comprend de la glycérine.

4. Produit cosmétique solide de maquillage selon l'une quelconque des Revendications 1 à 3 contenant 10 % en masse ou plus d'un composant huileux à base de silicone par rapport à la quantité totale du produit cosmétique.

5. Produit cosmétique solide de maquillage selon l'une quelconque des Revendications 1 à 4 qui est un fond de teint.
